# EUROPEAN PATENT SPECIFICATION

(11) **EP 4 284 265 B1**
(45) Date of publication and mention of the grant of the patent: **18.11.2026**
(21) Application number: 22705250.3
(22) Date of filing: 31.01.2022
(51) Int. Cl.: A61B 17/16, A61B 17/92

(54) **TRI-ROLL THREAD ELECTRIC SURGICAL IMPACT TOOL**
ELEKTROCHIRURGISCHES SCHLAGWERKZEUG MIT DREI ROLLENGEWINDE
OUTIL CHIRURGICAL ÉLECTRIQUE À PERCUSSION FILETÉ À TROIS ROULEAUX

(30) Priority: 01.02.2021 US 202163144285 P
(43) Date of publication of application: 06.12.2023
(73) Proprietor: Zimmer, Inc., Warsaw, Indiana 46580 (US)
(72) Inventor: SLOCUM, Alexander, Bow, New Hampshire 03304 (US)
(74) Representative: Taor, Simon Edward William
(86) International application number: PCT/US2022/014596
(87) International publication number: WO 2022/165357

(56) References cited:
- WO-A1-2018/044348
- WO-A1-2018/217250

## Description

### FIELD OF THE DISCLOSURE

The present disclosure relates generally to surgical instruments More specifically, the present disclosure relates to roll thread electric surgical impact tools.

### BACKGROUND

Orthopedic surgeons commonly utilize tools for cutting or carving bone that require a hammer or mallet to transmit an impaction force to the tool. An example is a broach tool used to prepare the proximal end of a femur to receive the stem of a hip implant. Such broaches can be used with a hammer wielded by the physician or with a pneumatic "jackhammer" like tool. However, striking a broach tool with a hammer can be tiresome and can cause high stresses on the physician's own joints, such as the shoulder joint. Furthermore, pneumatic impact tools require connection to an air hose, which can be inconvenient and can potentially limit the physician's ability to orient the tool in the desired manner.

WO2018044348A1 discloses a motor-driven orthopedic impacting tool for orthopedic impacting in the hips, knees, shoulders and the like. The tool is capable of holding a broach, chisel, or other end effector, which when gently tapped in a cavity with controlled percussive impacts, can expand the size or volume of an opening of the cavity or facilitate removal of the broach, implant, or other surgical implement from the opening. A stored-energy drive mechanism stores potential energy and then releases it to launch a launched mass or striker to communicate a striking force to an adapter in either a forward or reverse direction. The tool may further include a combination anvil and adapter and an energy adjustment mechanism to adjust the striking force the launched mass delivers to the adapter in accordance with a patient profile.

### SUMMARY

The following, non-limiting examples, detail certain aspects of the present subject matter to solve the challenges and provide the benefits discussed herein, among others.

According to an aspect, there is provided a rot surgical impact tool as set out in claim 1. Optional features are set out in the dependent claims.

### BRIEF DESCRIPTION OF THE FIGURES

In the drawings, which are not necessarily drawn to scale, like numerals can describe similar components in different views. Like numerals having different letter suffixes can represent different instances of similar components. The drawings illustrate generally, by way of example, but not by way of limitation, various embodiments discussed in the present document.
FIG. 1 shows a perspective view of a tri-roll thread electric surgical impact tool consistent with at least one example of this disclosure.
FIG. 2 shows a top cutaway view of a tri-roll thread electric surgical impact tool consistent with at least one example of this disclosure.
FIG. 3 shows a perspective view of a spring element subassembly of a tri-roll thread electric surgical impact tool consistent with at least one example of this disclosure.
FIG. 4 shows a cutaway view of a spring element subassembly of a tri-roll thread electric surgical impact tool consistent with at least one example of this disclosure.
FIG. 5 shows a perspective view of core elements of a spring element subassembly of a tri-roll thread electric surgical impact tool consistent with at least one example of this disclosure.
FIG. 6 shows an end plan view of a tri-roller subassembly consistent with at least one example of this disclosure.
FIG. 7 shows a perspective view of a tri-roller subassembly consistent with at least one example of this disclosure.
FIG. 8 shows a flowchart of logic usable for controlling a tri-roll thread electric surgical impact tool consistent with at least one example of this disclosure.
FIG. 9 shows a schematic of a controller consistent with at least one example of this disclosure.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

As an alternative to a pneumatic piston driven system, disclosed herein are electrically driven systems. Specifically, the electric surgical impact tools disclosed herein can include a motor operatively connected to a roller assembly operatively. The roller assembly can include a plurality of rollers and each of the plurality of rollers can be supported by a respective one of a plurality of roller arms. An impact structure can define a spring and an impact cavity that can include forward and reverse impact surfaces disposed on opposite ends of the impact cavity. The impact structure can include a wall comprising a plurality of thread elements connected to the wall and engageable with the roller assembly. Each of the thread elements can include a pair of helical end surfaces and a pair of axially extending surfaces that are engaged by the plurality of rollers. An impact flange of a tool holder element can be disposed within the impact cavity in between the forward and reverse impact surfaces.

During use, rotation of the roller assembly against the helical ends of the thread elements can cause axial movement of the impact structure to apply a load on the spring. Also, during use, engagement of the roller assembly against the axially extending surfaces of the thread elements can allow the spring to be unloaded and cause the impact structure to engage the impact flange.

As disclosed herein, in a forward impact mode the impact flange of the tool holder is moveable toward a rear end of the impact cavity when the tool implement is pressed against a workpiece (e.g., bone) and in a rearward impact mode the impact flange of the tool holder is movable toward a forward end of the impact cavity when the impact tool is pulled away from the workpiece. The forward impact mode can include rotating the roller assembly in a first direction against first helical ends of the thread elements and the rearward impact mode can include rotating the roller assembly in a second direction opposite the first direction and against second helical ends of the thread elements.

The surgical impact tools can include a housing supporting the impact structure and the tool holder. The housing can include a handle and a base. A trigger can be disposed in the handle and a battery can be connected to the base. A microphone and a controller can be disposed in the housing. The controller can be operative to control operation of the impact tool by voice command.

The above discussion is intended to provide an overview of subject matter of the present patent application. It is not intended to provide an exclusive or exhaustive explanation of the invention. The description below is included to provide further information about the present patent application.

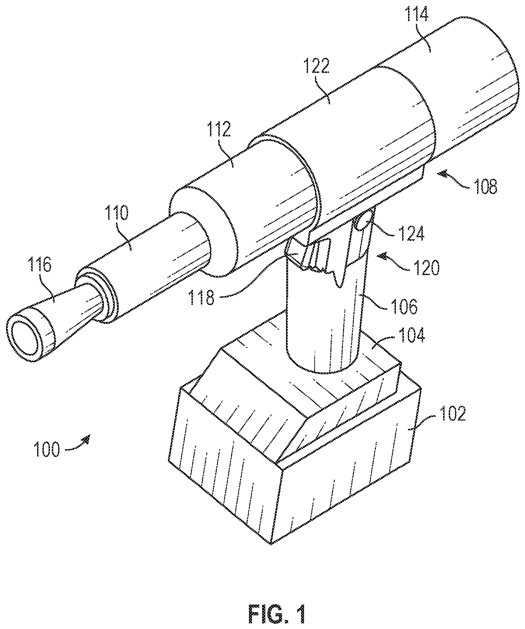

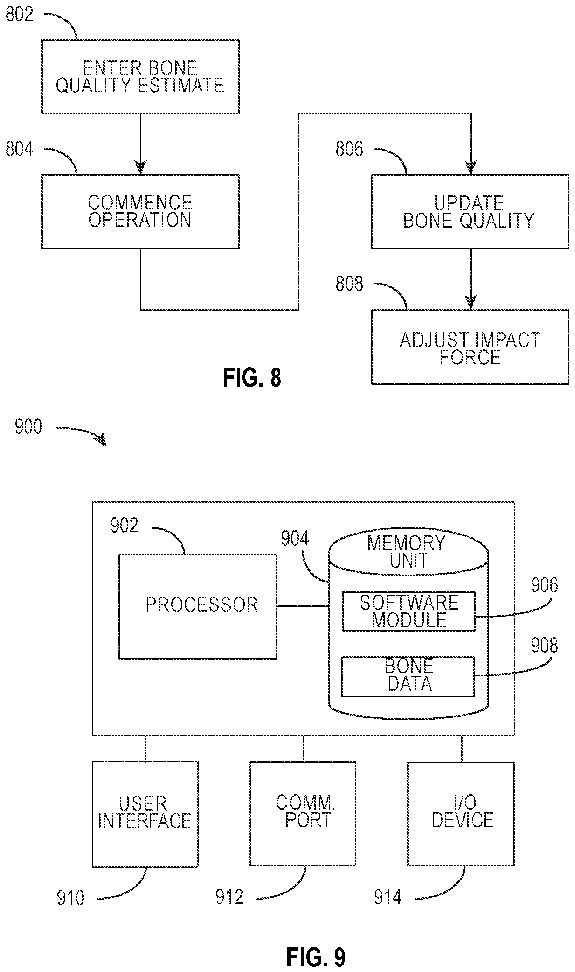

Turning now to the figures, FIG. 1 shows an example of a tri-roll thread electric surgical impact tool 100 consistent with at least one example of this disclosure. As disclosed herein, the tri-roll thread electric surgical impact tool 100, which can be cordless, can include a replaceable rechargeable battery pack 102 attached to a base 104, which can power and control electronics, such as controller 900 shown and describe below with respect to FIG. 9. A handle 106 can contain wires connected to a motor and/or sensors inside a main housing 108. A trigger 118 can provide manual on-off control and a speaker/microphone 124 can allow the user to speak instructions and hear suggestions from a system controller as disclosed herein. In other words, speaker/microphone 124 can allow the tri-roll thread electric surgical impact tool 100 to be controlled by voice commands. A handle system 120 can be attached to the main housing 108 by structure 122. The main housing 108 can include a distal portion 114, a middle portion 112, and a proximal portion 110. An orthopedic or other tool can be held in a tool holder 116.

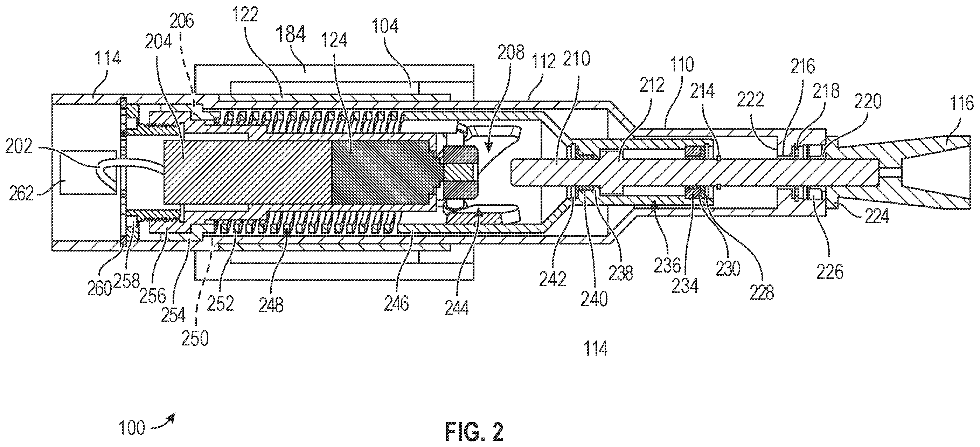

FIG. 2 shows a cross section from a top of the tri-roll thread electric surgical impact tool 100. The impact tool 100 can include a machined bi-directional (e.g., tension or compression) spring structure 248 with spring coils 252, shown in a neutral position in FIG. 2, and a tri-thread 244 actuated by a tri-roller 208 that can be attached directly to an output shaft of a gearbox 124. A motor 204 can be attached to the gearbox 124. For example, the gearbox 124 can be a ball-bearing-mini-motor-gearhead with a 62:1 ratio and can be used to provide 4 Joules of impact energy.

The motor 204 can have a power/position sensor data cable 202 that can extend out the back and down through a slot 262 in the distal outer housing section 114. An internal snap ring 260 can serve as a structure that a nut 258 can push against as it forces forward the motor housing structure 256. A step 206 on the motor housing structure 256 can push on a mating step 250 on the machined spring structure 254, which in turn can push on the step transition between the outer housing sections 114 and 112. The faces of these the steps 206 and 250 can all be machined perpendicular to a longitudinal axis such that radial misalignment between them at their proximal ends can be less than 0.1 mm. With an axial/diameter dimension ratio of about 5:1 this can produce a squareness requirement readily achievable for parts machined on a precision lathe.

To create a forward impact, a user can press the impact tool 100 forward so a distal face of flange 224 of tool holder 116 presses against a proximal face 226 of the structure 110. A forward portion 246 of the machined spring structure 248 can hold the tri-lobe thread elements (three) 244, which can be engaged by the tri-roller (three rollers) 208 that can be attached to the output shaft of the gearbox 124. When the shaft of the gearbox 124 rotates in one direction, the engagement of the rollers 208 with the threads 244 can cause the machined spring 248 to compress and draw backwards to store energy. A base 256 of the spring 248 can be fixed and not rotate due to a tight preload against the structure 108 by the nut 258.

As the spring 248 compresses, the proximal end 236 of the machined spring structure 248 can move distally. The proximal end 236 of the spring structure 248 can contain a snap ring 242 that can hold a flanged sliding contact bushing bearing 240 (e.g., a Rulon bearing) in place. The bushing bearing 240 can have an inner diameter of, for example, about 10mm, which can slide over a shaft of a tool holder element 210. The proximal end of the tool holder element 210 can be guided by a flanged bushing bearing 216 that can be held in place by a snap ring 218. A sliding seal 220 can keep foreign matter out. When a striker flange 238 of the spring structure 248 reaches its fully distally retracted position, it can be then released by the tri-roller 208 reaching an end of travel and the turnaround portion of the tri-thread 244, to rapidly accelerate and cause proximal face of 238 to strike a distal face of the flange 212 on the tool holder element 210. Continued operation of the motor 204 of the impact tool 100 while the user is pushing forward on the impact tool 100 can repeat the process to drive a tool implement more and more into a work piece (e.g., broach cutting into a femur).

To enable assembly and to retract the tool, the machined spring structure 248 can have a collar 234 with bearing 230 and both are held in place by a snap ring 242. A snap ring 214 on the tool holder element 210 can move as the user pulls back on the impact tool 100 until it contacts a surface 222 and the flange 212 can approach the distal surface of collar 234. The motor 204 can then be operated in a reverse direction and the tri-roller 208 can engage the reversing thread surfaces of tri-thread 244 to extend the machined spring 252 to move the proximal structure 236 forward so collar 234 moves proximally away from collar 212. At the end of the thread engagement between tri-roller 208 and tri-threads 244, the spring 248 can snap back to deliver a retract blow between the distal face of collar 234 and the proximal face of flange 212. As the user continues pulling back on the impact tool 100 the motor rotation can repeat the process.

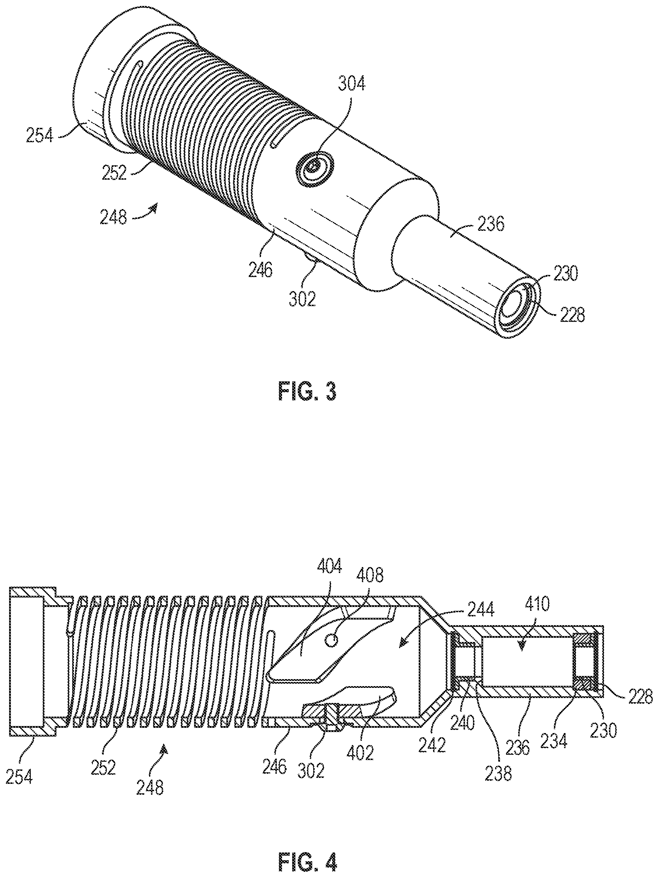

FIGS. 3 and 4 show the tri-spring threads/spring subassembly comprising the spring structure 248, the tri-threads 244, and bearings 240 and 230 held in place by snap rings 242 and 228, respectively. The impact cavity 77, sometimes called an impact chamber, can be bound on distal end by striker flange 238 and the proximal end by the collar 234, where the flange 212 of the tool holder element 210 can move back and forth in the impact cavity 77.

The spring coils 252 can be machined in the spring structure 248 so they are monolithic, and three coils 252 can be used to give the system greater lateral stability. The spring structure 248 can be made from a high strength stainless steel or a high strength steel such as a maraging steel (e.g., Aeromet 340). The tri-threads 244 can be made as inserts 506, 402, and 404 so that they can be precisely manufactured, hardened, and precision ground. The tri-threads 244 can then be attached by high strength flanged screws 302, 304, and 408, respectively. The tri-thread inserts oblong shape can mean that when held against the inside surface of the cylinder structure 246 they can resist twisting moments, but they can also be bonded in place in addition to being bolted by screws 302, 304, and-408 as insurance against loosening in the impact environment.

One manufacturing method that can be used to make the machined spring coils 252 integral with the spring structure 248 is to machine all but the spring coils 252 using machining operations, such as on a lathe. Then, an internal close fitting solid cylindrical mandrel can be inserted that is temporarily attached using a low melt alloy, such as those that have Gallium as a base metal. This can enable the spring coils 252 to be machined without risk of damage while machining.

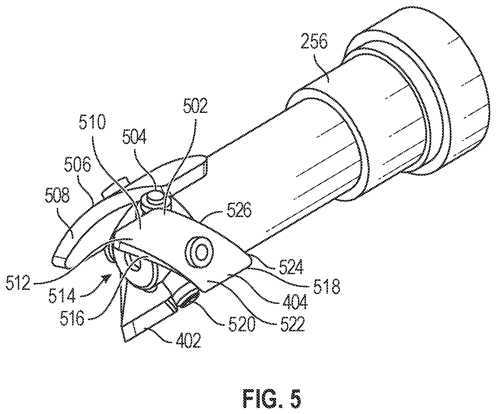

FIG. 5 shows the tri-threads 244 without the spring structure 248 to which they are attached. Here the tri-roller 208 can be seen with its three thread rollers 504, 618, and 520 on central tri-arm structure 514 that can have a D-bore typically to enable transmission of high torque, although a keyed connection can be made to a gearmotor output shaft. The rollers 504, 618, and-520 can be ball bearings or for very high loads, needle roller bearings, and can be held in place by snap rings. Each tri-thread element, such as 404, can be sized to have a helical proximal forcing contact surface 502 and a helical distal forcing contact surface 522 for contact with a respective thread roller 520. For example, looking distally, as the motor gearbox 124 rotates the tri-roller 208 counterclockwise, a roller such as 520 can contact surface 516 compressing the springs 252 until the roller520 gets to the radiused end 512. Axially straight surface 510 means that the spring can then thrust the proximal end 246 and 236 with impact chamber 410 in the proximal direction as the tri-roller continues to rotate counterclockwise can then come to contact helical thread surface 508 and the process can continue so multiple rapid strikes can be made. If the tri-roller 208 rotates clockwise, then corresponding opposite elements can be engaged, such as distal helical thread 526 to extend the springs 252 so when the roller 520 comes to the end of a thread and rolls around end 524 for example, the springs 252 can snap the proximal end 246 and 236 back in the distal direction for causing the collar 234 to impact the flange 212 of the tool holder element 210 for creating reverse impacts to aid in extraction of the tool implement.

The circumferential space between the helical thread surfaces and corresponding axially straight surfaces can be the diameter of a roller plus the distance travelled in the time it takes the spring to return while the motor keeps rotating. This can be only a few millimeters and can be determined based on the moving mass spring constant and spring compression.

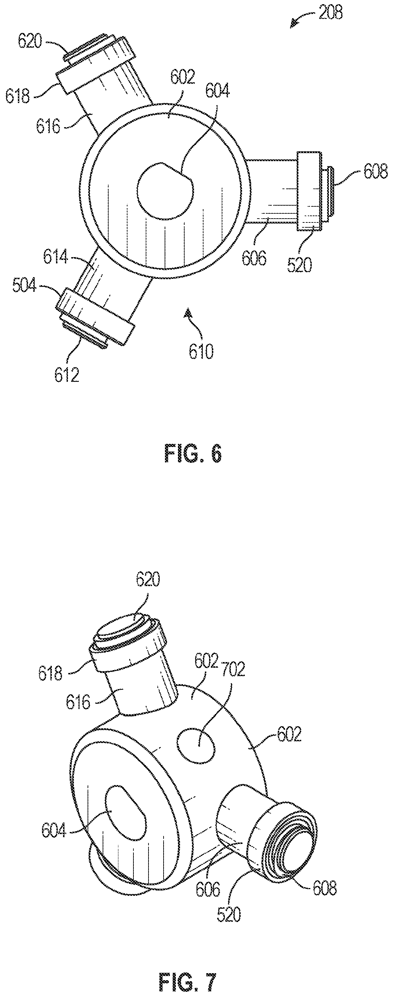

FIGS. 5, 6, and 7 show the tri-roller subassembly 208 with its rollers 504, 618, and 520 on arms 614, 616, 606, which can protrude from central body 602 with D-shaped bore 604. Snap rings 612, 620, and 608 can hold their respective bearings in place. A set screw can be threaded into the threaded aperture 702 to axially secure the tri-roll subassembly 208 to the shaft of the motor gearbox 124 with a dimple on the shaft flat. The arms and body of the tri-roller subassembly 208 can be machined, or printed, or integrally molded to be integral (solid) for maximum strength when forming the tri-arm body 610.

Three arms and rollers 504, 618, and-520, and corresponding threads 506, 402, and-404, can provide radial stability while transmitting torque from the motor gearbox 124 without creating radial loads on the output shaft of the gearbox 124. In addition, the contact forces can be less than or equal to the torque from the gearbox 124 divided by the product of three and the radial distance from the roller center to the rotating shaft center. The rolling contact between the rollers 504, 618, and-520 and the thread elements 506, 402, and-404 can reduce contact stresses and wear, such as in the rounded end transition regions between the helical thread surfaces 508 and516and the axial return regions 510 and 518.

The roller assembly 208 include a hardened, ground tapered roller rotating in sealed, lifetime-lubricated ball bearings. Coupled with the use of dry lubricant surfaces in the thread grooves, this lifetime-lubrication can result in virtually maintenance-free operation. This is not a cam system which inherently has a varying gain (transmission ratio) as a function of position; rather this is a screw thread that has a constant transmission ratio, which means it responds in a linear manner, until it reverses.

The present disclosure herein combines the concept of a rollnut with the reversing screwdriver thread that instead of the threads crossing over themselves, the contact surfaces can be opposed and the motor can be reversed to reverse the direction. However, continuous motor rotation in either direction can result in continual oscillation with either a proximal or distal oscillation bias. Instead of a rotating threaded nut surrounding a screw shaft, the present disclosure uses a female thread that can be prevented from rotation but allowed to move axially, thus forming an internal threaded sleeve.

Engaging the threads can be an inside out tri-start rollnut where a tri-roller structure can be attached to the gearmotor output shaft. At the end of each arm of the tri-roller 208 can be a ball bearing with a convex (curved) profile outer race (either specially manufactured or a sleeve made and shrunk or bonded to the bearing outer ring). By using three rollers 504, 618, and-520 to engage the three internal threads 506, 402, and-404 of the coupler 248, the coupler 248 can be centered about the gearmotor axis of rotation and there can be no net radial loads on the gearbox shaft. The tri-roller 208 can experience axial loads and thus the gearmotor output shaft can have a snap ring groove to ensure the tri-roller 208 does not work its way off the shaft.

The tri-threads 506, 402, and-404 can be cut from one piece or individual inserts, which can be made in high volume using a powder metallurgy technique. The surfaces that the rollers 504, 618, and-520 can be contact finish ground after hardening. When cut with the center of an end mill tracing the helix, the contact surfaces will not be aligned with the longitudinal axis of the spiral. Furthermore, the outer rings of the bearing on the tri-roller arm ends 612, 620, and-608 are also not aligned perfectly with the longitudinal axis. Hence a slight taper of the surfaces (or end mill or grinding tool used to cut them) might be needed, such as, for example, a 1 degree taper. Furthermore, at the turn around zone at the end of travel, there can be a further mismatch potential between the roller and the thread turn around surface, which can be adjusted to ensure line contact. Note the differential radius can imply slipping and hence once again a crowned roller can be used.

The spring structure 248 can be machined from a tubular structure, and a triple start thread 252 can be used to help maximize lateral stability. A machined spring thread can be bi-directional and thus reloaded. Hence only one spring 248 can be needed for storing and releasing energy in either the forward or reverse motion for driving or extracting a tool respectively.

FIG. 8 shows a method for controlling a tri-roll thread electric surgical impact tool, such as impact tool 100, consistent with at least one example of this disclose. During and/or before a surgical procedure, a surgeon can enter an estimate of bone quality into a controller (802), the operation may commence (804). For example, bone quality scores can range from 1, poor quality bone, to 5 for high quality bone. Depending on the bone quality the tool may be set to deliver a predetermine impact force. For example, for low bone quality a low impact force can be set. For a higher bone quality, a higher impact force can be set.

During the operation, the bone quality can be updated (806), using a tool/position sensor sensing, based on how quick the tool is moving into the bone on the first few broaches. For example, if the broach is sliding in faster than expected due to weak cancellous bone (e.g., osteoporosis), the bone quality can be updated. The goal of the initial bone quality assessment can be to modulate the starting force (initial impact) and adjust the amount of subsequent impact modulation as the tool progresses into the bone (808). As the tool keeps impacting as broaches are increased in size, for example, the energy is monitored and remains unchanged when there is "maximum" broach movement down the femur canal (as measured by the position sensor) for example.

Updating the bone quality can be a continuous process. For example, as the position sensor notes that the broach or implant is not advancing forward as much ("medium movement forward"), which can indicate the end of travel. Continuing to impact the bone harder may damage the bone so the tool can automatically modulate the force down a specific percentage (e.g., a more significant decrease for weaker bone, less significant for stronger bone, etc.

FIG. 9 shows an example schematic of controller 900, in accordance with at least one example of this disclosure. As shown in FIG. 9, the controller 900 can include a processor 902 and a memory 904. The memory unit 904 can include a software module 906 and bone data 908. While executing on the processor 902, the software module 906 can perform processes receiving displacement data, determining bone quality, adjusting an impact force of a tool, etc., including, for example, one or more stages included in the method described herein.

As disclosed herein, the bone data 1208 can include formulas, lookup tables, actuarial tables, patient data, etc. that can be used to determine bone quality as disclosed herein. The bone data 1208 can also include data for correlating impact forces desirable for given bone qualities and for various sizing of tools, such as rasps and/or broaches. The controller 900 can also include a user interface 910, a communications port 912, and an input/output (I/O) device 914.

The user interface 910 can include any number of devices that allow a user to interface with the controller 900. Non-limiting examples of the user interface 910 can include a keypad, such as buttons located on a housing of a linear electric surgical hammer impact tool, a microphone, a display (touchscreen or otherwise and connected to controller via a wired or wireless connection), etc.

As an example, a surgeon, as he or she is observing the impact, can speak "less force," "more force," "faster," "slower," etc. and it will be understood that the impact tool 100 can be controlled by the user to meet their needs and style of use using his or her voice. The controller 900 can learn to respond to a particular user.

For the controller 900 to obtain less force it can control the distance of acceleration and current to a coil structure to vary the force and hence the acceleration of the impact element. The impact force can be proportional to energy, which is the product of one half the mass of the impact element and its velocity squared. "Faster" spoken by the user can mean that the controller 900 would cause the impact element to reciprocate back and forth faster. The controller 900 can operate in current control mode, to generate the desired force, and the voltage follows according to the speed as the impact element accelerates.

The communications port 912 may allow the controller 900 to communicate with various information sources and devices, such as, but not limited to, remote computing devices such as servers or other remote computers. For example, remote computing devices may maintain data, such as patient scan data, that can be retrieved by the controller 900 using the communications port 912. Non-limiting examples of the communications port 912 can include, Ethernet cards (wireless or wired), Bluetooth^{®} transmitters and receivers, near-field communications modules, etc.

The I/O device 914 can allow the controller 900 to receive and output information. Non-limiting examples of the I/O device 914 can include, sensors, such as Hall effect sensors, a camera (still or video), a microphone, etc. For example, the I/O device 914 can allow the controller 900 to directly receive patient data from a CT scanning device, x-ray machine, etc. As another example, the I/O device 914 can include a Hall effect sensor that transmits one or more signals received by the processor 902. The processor 902 can then determine a position of the tri-roller element and/or an impact force to be generated by the tri-roller based on the position of the tri- roller.

### NOTES

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) can be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features can be grouped together to streamline the disclosure. This should not be interpreted as intending that an unclaimed disclosed feature is essential to any claim. Rather, inventive subject matter can lie in less than all features of a particular disclosed embodiment. The scope of the invention is determined by the appended claims.

## Claims

1. A surgical impact tool comprising:
a motor (204);
a roller assembly operatively connected to the motor (204) and comprising a plurality of rollers (208), each of the plurality of rollers (208) supported by a respective one of a plurality of roller arms;
an impact structure defining a spring (248) and an impact cavity (77) including forward and reverse impact surfaces disposed on opposite ends of the impact cavity (77), the impact structure comprising a wall comprising a plurality of thread elements (244) connected to the wall and engageable with the roller assembly, each of the thread elements (244) including a pair of helical end surfaces and a pair of axially extending surfaces that are engaged by the plurality of rollers (208);
a tool holder (210) comprising an impact flange disposed within the impact cavity (77) in between the forward and reverse impact surfaces; and
wherein, during use:
rotation of the roller assembly against the helical end surfaces of the thread elements (244) causes axial movement of the impact structure to apply a load on the spring (248), and
engagement of the roller assembly against the pair of axially extending surfaces of the thread elements (244) allows the spring (248) to be unloaded and cause the impact structure to engage the impact flange.

2. The surgical impact tool of claim 1, wherein in a forward impact mode the impact flange of the tool holder (210) is movable toward a rear end of the impact cavity (77) when a tool implement is pressed against a workpiece and in a rearward impact mode the impact flange of the tool holder (210) is movable toward a forward end of the impact cavity (77) when the impact tool is pulled away from the workpiece.

3. The surgical impact tool of claim 2, wherein the forward impact mode includes rotating the roller assembly in a first direction against first helical ends of the thread elements (244) and the rearward impact mode includes rotating the roller assembly in a second direction opposite the first direction and against second helical ends of the thread elements (244).

4. The surgical impact tool of any one of claims 1-3, wherein the spring (248) is formed integral with the wall.

5. The surgical impact tool of any one of claims 1-4, wherein the spring (248) includes at least three coil springs.

6. The surgical impact tool of any one of claims 1-5, wherein the thread elements (244) are connected to the wall by screws.

7. The surgical impact tool of any one of claims 1-6, further comprising a gearbox (124) connected to the motor (204).

8. The surgical impact tool of claim 7, wherein the gearbox (124) and the motor (204) are disposed within the spring (248) of the impact structure.

9. The surgical impact tool of any one of claims 1-8, further comprising:
a housing (108) supporting the impact structure and the tool holder (210), the housing (108) comprising a handle (106) and a base (104);
a trigger (118) disposed in the handle (106); and
a battery (102) connected to the base (104).

10. The surgical impact tool of claim 9, further comprising:
a microphone (124) disposed in the housing; and
a controller (900) operative to control operation of the impact tool by voice command.

## Patentansprüche

1. Chirurgisches Schlagwerkzeug, umfassend:
einen Motor (204);
eine Rollenbaugruppe, die mit dem Motor (204) wirkverbunden ist und eine Vielzahl von Rollen (208) umfasst, wobei jede der Vielzahl von Rollen (208) durch einen jeweiligen einer Vielzahl von Rollenhebeln getragen wird;
eine Schlagstruktur, die eine Feder (248) und einen Schlaghohlraum (77) definiert, der eine Vorwärts- und eine Rückwärtsschlagoberfläche einschließt, die an gegenüberliegenden Enden des Schlaghohlraums (77) angeordnet sind, wobei die Schlagstruktur eine Wand umfasst, die eine Vielzahl von Gewindeelementen (244) umfasst, die mit der Wand verbunden ist und mit der Rollenbaugruppe in Eingriff bringbar ist, wobei jedes der Gewindeelemente (244) ein Paar von spiralförmigen Endoberflächen und ein Paar von sich axial erstreckenden Oberflächen einschließt, die durch die Vielzahl von Rollen (208) in Eingriff genommen sind;
einen Werkzeughalter (210), der einen Schlagflansch umfasst, der innerhalb des Schlaghohlraums (77) zwischen der Vorwärts- und der Rückwärtsschlagoberfläche angeordnet ist; und
wobei während der Verwendung:
eine Drehung der Rollenbaugruppe gegen die spiralförmigen Endoberflächen der Gewindeelemente (244) bewirkt, dass eine axiale Bewegung der Schlagstruktur eine Last auf die Feder (248) aufbringt, und
ein Eingriff der Rollenbaugruppe gegen das Paar von sich axial erstreckenden Oberflächen der Gewindeelemente (244) zulässt, dass die Feder (248) entlastet wird und bewirkt, dass die Schlagstruktur den Schlagflansch in Eingriff nimmt.

2. Chirurgisches Schlagwerkzeug nach Anspruch 1, wobei in einem Vorwärtsschlagmodus der Schlagflansch des Werkzeughalters (210) zu einem hinteren Ende des Schlaghohlraums (77) hin bewegbar ist, wenn ein Werkzeugmittel gegen ein Werkstück gedrückt wird, und in einem Rückwärtsschlagmodus der Schlagflansch des Werkzeughalters (210) zu einem vorderen Ende des Schlaghohlraums (77) hin bewegbar ist, wenn das Schlagwerkzeug von dem Werkstück weggezogen wird.

3. Chirurgisches Schlagwerkzeug nach Anspruch 2, wobei der Vorwärtsschlagmodus Drehen der Rollenbaugruppe in einer ersten Richtung gegen erste spiralförmige Enden der Gewindeelemente (244) einschließt und der Rückwärtsschlagmodus Drehen der Rollenbaugruppe in einer zweiten Richtung entgegengesetzt zu der ersten Richtung und gegen zweite spiralförmige Enden der Gewindeelemente (244) einschließt.

4. Chirurgisches Schlagwerkzeug nach einem der Ansprüche 1-3, wobei die Feder (248) einstückig mit der Wand ausgebildet ist.

5. Chirurgisches Schlagwerkzeug nach einem der Ansprüche 1-4, wobei die Feder (248) mindestens drei Schraubenfedern einschließt.

6. Chirurgisches Schlagwerkzeug nach einem der Ansprüche 1-5, wobei die Gewindeelemente (244) durch Schrauben mit der Wand verbunden sind.

7. Chirurgisches Schlagwerkzeug nach einem der Ansprüche 1-6, ferner umfassend ein Getriebe (124), das mit dem Motor (204) verbunden ist.

8. Chirurgisches Schlagwerkzeug nach Anspruch 7, wobei das Getriebe (124) und der Motor (204) innerhalb der Feder (248) der Schlagstruktur angeordnet sind.

9. Chirurgisches Schlagwerkzeug nach einem der Ansprüche 1-8, ferner umfassend:
ein Gehäuse (108), das die Schlagstruktur und den Werkzeughalter (210) trägt, wobei das Gehäuse (108) einen Griff (106) und eine Basis (104) umfasst;
einen Auslöser (118), der in dem Griff (106) angeordnet ist; und
eine Batterie (102), die mit der Basis (104) verbunden ist.

10. Chirurgisches Schlagwerkzeug nach Anspruch 9, ferner umfassend:
ein Mikrofon (124), das in dem Gehäuse angeordnet ist; und
eine Steuerung (900), die wirksam ist, um einen Betrieb des Schlagwerkzeugs durch Sprachbefehl zu steuern.

## Revendications

1. Outil chirurgical à percussion comprenant :
un moteur (204) ;
un ensemble de rouleaux relié de manière fonctionnelle au moteur (204) et comprenant une pluralité de rouleaux (208), chacun de la pluralité de rouleaux (208) étant supporté par un bras respectif d'une pluralité de bras de rouleau ;
une structure de percussion définissant un ressort (248) et une cavité de percussion (77) comprenant des surfaces de percussion avant et inverse disposées sur les extrémités opposées de la cavité de percussion (77), la structure de percussion comprenant une paroi comprenant une pluralité d'éléments filetés (244) reliés à la paroi et pouvant se mettre en prise avec l'ensemble de rouleaux, chacun des éléments filetés (244) comprenant deux surfaces d'extrémité hélicoïdales et deux surfaces s'étendant axialement qui sont mises en prise par la pluralité de rouleaux (208) ;
un porte-outil (210) comprenant une bride de percussion disposée à l'intérieur de la cavité de percussion (77) entre les surfaces de percussion avant et arrière ; et
lors de l'utilisation :
ladite rotation de l'ensemble de rouleaux contre les surfaces d'extrémité hélicoïdales des éléments filetés (244) provoquant le déplacement axial de la structure de percussion de manière à appliquer une charge sur le ressort (248), et
ladite mise en prise de l'ensemble de rouleaux contre les deux surfaces s'étendant axialement des éléments filetés (244) permettant au ressort (248) d'être relâché et d'amener la structure de percussion à se mettre en prise avec la bride de percussion.

2. Outil chirurgical à percussion selon la revendication 1, dans un mode de percussion avant, ladite bride de percussion du porte-outil (210) étant mobile vers une extrémité arrière de la cavité de percussion (77) lorsqu'un outil est pressé contre une pièce à travailler et, dans un mode de percussion arrière, ladite bride de percussion du porte-outil (210) étant mobile vers une extrémité avant de la cavité de percussion (77) lorsque l'outil à percussion est éloigné de la pièce à travailler.

3. Outil chirurgical à percussion selon la revendication 2, ledit mode de percussion avant comprenant la rotation de l'ensemble de rouleaux dans un premier sens contre les premières extrémités hélicoïdales des éléments filetés (244) et ledit mode de percussion arrière comprenant la rotation de l'ensemble de rouleaux dans un second sens opposé au premier sens et contre les secondes extrémités hélicoïdales des éléments filetés (244).

4. Outil chirurgical à percussion selon l'une quelconque des revendications 1 à 3, ledit ressort (248) étant formé d'un seul tenant avec la paroi.

5. Outil chirurgical à percussion selon l'une quelconque des revendications 1 à 4, ledit ressort (248) comprenant au moins trois ressorts hélicoïdaux.

6. Outil chirurgical à percussion selon l'une quelconque des revendications 1 à 5, lesdits éléments filetés (244) étant reliés à la paroi par des vis.

7. Outil chirurgical à percussion selon l'une quelconque des revendications 1 à 6, comprenant en outre une boîte de transmission (124) reliée au moteur (204).

8. Outil chirurgical à percussion selon la revendication 7, ladite boîte de transmission (124) et ledit moteur (204) étant disposés à l'intérieur du ressort (248) de la structure de percussion.

9. Outil chirurgical à percussion selon l'une quelconque des revendications 1 à 8, comprenant en outre :
un boîtier (108) supportant la structure de percussion et le porte-outil (210), le boîtier (108) comprenant une poignée (106) et une base (104) ;
une gâchette (118) disposée dans la poignée (106) ; et
une batterie (102) reliée à la base (104).

10. Outil chirurgical à percussion selon la revendication 9, comprenant en outre :
un microphone (124) disposé dans le boîtier ; et
un dispositif de commande (900) servant à commander le fonctionnement de l'outil à percussion par commande vocale.
